(19) **Europäisches Patentamt European Patent Office Office européen des brevets**

(11) **EP 4 685 484 A1**

(12) **EUROPEAN PATENT APPLICATION**

(43) Date of publication:
**28.01.2026 Bulletin 2026/05**

(21) Application number: **24191023.1**

(22) Date of filing: **25.07.2024**

(51) International Patent Classification (IPC):
**G01N 33/50** (2006.01)

(52) Cooperative Patent Classification (CPC):
**G01N 33/5073**; G01N 2333/4728; G01N 2800/325

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Target Cells Spólka z ograniczona odpowiedzialnoscia**
**31-545 Kraków (PL)**

(72) Inventors:
• **Zuba-Surma, Ewa**
**30-363 Kraków (PL)**

• **Karnas, Elzbieta**
**30-438 Kraków (PL)**
• **Dudek, Patrycja**
**30-716 Kraków (PL)**
• **Phillips, Catherine**
**Grayslake, 60030 (US)**
• **McWherter, Robin**
**Third Lake, 60030 (US)**

(74) Representative: **Krekora, Magdalena**
**ul. Gorna 95**
**PL-32-091 Michalowice (PL)**

(54) **METHOD, ASSAY AND USE OF ALPHA-1-ACID GLYCOPROTEIN FOR DETERMINING THE BINDING CAPACITY OF STEM CELLS**

(57) A method for determining the binding capacity of stem cells to mammalian heart tissue consists in that: (a) in the well matrix, some of the wells are filled with $\alpha$-1 acid glycoprotein solution and incubated; (b) after incubation, all wells are filled with a suspension of stem cells labeled with a fluorescent dye and incubated; (c) after incubation, the whole is washed; (d) the fluorescence level is then measured. The object of the invention is also an assay and use of $\alpha$-1 acid glycoprotein to determine the binding capacity of stem cells.

Fig. 2

EP 4 685 484 A1

**Description**

TECHNICAL FIELD

Method, assay and use of $\alpha$-1 acid glycoprotein for determining the binding capacity of stem cells to heart tissue of a mammal, in particular human.

BACKGROUND ART

Stem cells (SC) are cells that have the ability to self-renew, i.e. maintain their pool, as well as differentiate into cells of various types that build individual tissues and organs.

Mesenchymal stem cells (MSC) are multipotent SC, i.e. capable of differentiating into different cell types within one germ layer - i.e. mesoderm, giving rise to progenitors that create, among others, bone, cartilage and adipose tissue. Importantly, thanks to their paracrine and immunomodulatory properties, MSCs participate in the regeneration of damaged tissues, stimulating cells residing in the niche to undertake reparatory activity, remodeling the extracellular matrix and silencing inflammation. Cells with MSC characteristics have been isolated from several organs and tissues of the human body, including bone marrow, adipose tissue, umbilical cord, skin, skeletal muscles, dental pulp and many others. The relative ease of isolation and in vitro culture make them an interesting research target for potential use in regenerative medicine, including the treatment of heart diseases.

Cardiovascular diseases (CVDs) are significant health problem that affects millions of patients around the world and they are one of the most common causes of hospitalization. Mortality among patients is still high and reaches up to approx. 10% regardless of etiology. One of the main causes of CVDs is an acute myocardial infarction (acute coronary syndrome). As existing therapies are still not satisfactory, possibility of use of SC in the treatment of heart diseases gives hope for the development of breakthrough therapies in this area.

On-going research and therapies require the use of preclinical tests. Thus, rapid *in vitro* tests are currently preferred, which additionally allows for reducing the number of tests performed on animals.

Patent US7575938B2 reveals a way to quickly diagnose liver disease in its early stages. The method involves assessing the asialo $\alpha$-1 acid glycoprotein in a test sample using a monoclonal antibody.

From the patent EP3 816629B1 there is known a kit for specific determination of the degree of progressive chronic hepatitis and liver fibrosis based on a blood sample and an immunochromatographic strip, containing an HRP-RCA II conjugate (Ricinus communis II agglutinin) or a Gold-RCA II conjugate specifically binding to asialo $\alpha$-1 acid glycoprotein, wherein the monoclonal antibody AGP601 for asialo $\alpha$-1 acid glycoprotein produced by the fusion cell line deposited under the number KCTC13998BP and horseradish peroxidase (HRP) are self-linked or gold-linked nanoparticles.

From the patent application CN105942966A there is known a kit for detecting $\alpha$1-acid glycoprotein by latex immunoturbidimetry, which includes a solution of latex particles coupled with an antibody against human $\alpha$1-acid glycoprotein. The kit can be used to detect $\alpha$-1 acid glycoprotein in human urine.

Patent application EP1436615A1 discloses an assay for detecting parameters associated with a vascular disease including cardiovascular, stroke, pulmonary, renovascular, cerebrovascular, thrombotic or generalized arterial or venous condition or event including acute coronary syndrome such as but not limited to acute myocardial infarction, heart failure, atheromoma or a thrombotic condition.

Patent application WO0039590A3 discloses a method for determining the dosage of a taxoid to administer to a patient who is being treated for cancer and whose body fluids include $\alpha$-1-acid glycoprotein comprising observing the patient's level of $\alpha$-1 acid glycoprotein, evaluating said level to determine the dosage of the taxoid to administer to the patient by comparing said level to a predetermined $\alpha$-1 acid glycoprotein level derived from a population of patients having said cancer and treated with said taxoid at a common dosage and based on said evaluation, recommending the dosage of the taxoid to administer to the patient.

Patent US7358055B2 discloses methods for diagnosing and assessing acute coronary syndromes. Tested patient samples are analyzed for the presence and number of units of a panel of markers including one or more specific markers of myocardial damage and one or more non-specific markers of myocardial damage. Various markers have been disclosed for a panel of markers for such diagnosis and evaluation. In various aspects, the invention provides methods for the early detection and differentiation of stable angina, unstable angina and myocardial infarction. The methods of the invention provide rapid, sensitive, and specific tests that can significantly increase the number of patients who can receive favorable treatments and therapies, reduce costs associated with misdiagnosis, and provide important information about a patient's prognosis.

Patent US7282222B2 discloses methods of delivering cells to a target tissue in a mammal using a glycoconjugate to direct the cells to the desired organ in the mammal. The methods of the present invention are particularly suitable for the delivery of stem cells, such as those derived from bone marrow or umbilical cord tissue. The methods also include directing a gene of interest to a mammalian tissue by introducing a cell containing the gene of interest and administering the glycoconjugate to the mammal.

None of the known solutions provide *in vitro* assays that would enable determination of the ability of stem cells to bind to mammalian heart tissues.

DISCLOSURE OF THE INVENTION

A method for the determination of the binding capacity of stem cells to mammalian heart tissue according to the invention is characterized in that:

(a) in the well matrix, some of the wells are filled with $\alpha$-1 acid glycoprotein solution and incubated;

(b) after incubation, all wells are filled with a suspension of stem cells labeled with a fluorescent dye and incubated;

(c) after incubation, the whole is washed;

(d) the fluorescence level is then measured.

Preferably the mammal is a human.
Preferably the $\alpha$-1 acid glycoprotein solution is $\alpha$-1 acid glycoprotein suspended in a buffered saline solution.
Preferably the concentration of the $\alpha$-1 acid glycoprotein solution is from 1 $\mu$g/ml to 1 mg/ml.
Preferably the volume of the $\alpha$-1 acid glycoprotein solution in each filled well is from 10% to 100% of the volume of the well.
Preferably the matrix is incubated with a solution of $\alpha$-1 acid glycoprotein for a period of 10 min to 48 hours at a temperature of 2 to 40 degrees Celsius.
Preferably after the time of incubation of the matrix with the $\alpha$-1 acid glycoprotein solution, the $\alpha$-1 acid glycoprotein solution is washed out by washing the matrix with a liquid in a volume ranging from 10% to 100% of the volume of the matrix well.
Preferably after washing the matrix, it is dried for a period of 1 minute to 12 hours at a temperature of 2 to 40 degrees Celsius in sterile conditions, and then covered with a cover adapted to the size of the matrix.
Preferably the concentration of stem cells in the suspension is from 10,000 cells/ml to 1,000,000 cells/ml.
Preferably the incubation period of the matrix with the stem cell suspension is from 1 minute to 24 hours at a temperature from 2 to 40 degrees Celsius.
Preferably after incubation, stem cells unbound to the matrix are washed away by washing the matrix with a liquid in a volume from 10% to 100% of the volume of the matrix well.
Preferably the matrix with bound stem cells (after rinsing off unbound cells) is filled with liquid in a volume from 10% to 100% of the volume of each well of the matrix and fluorescence is measured.
The object of the present invention is also a diagnostic assay kit for the determination of the binding capacity of stem cells to mammalian heart tissue, characterized in that it comprises: a matrix with wells and an $\alpha$-1 acid glycoprotein.
Preferably the mammal is a human.
Additionally the object of the present invention is use of $\alpha$-1 acid glycoprotein to determine the binding capacity of stem cells to mammalian cardiac tissues.
Preferably the mammal is a human.
Preferably the use is characterized in that:

(a) the $\alpha$-1 acid glycoprotein solution is incubated;
(b) after incubation, a suspension of stem cells labeled with a fluorescent dye is added to the solution and incubated;
(c) after incubation, the whole is washed;
(d) the fluorescence level is then measured.

The term $\alpha$-1 acid glycoprotein (A1GP) includes any glycoform of the A1GP protein that may be represented by (S)-Gal-GlcNAc-Man-GlcNAc-P, where S is a terminal sugar, sialated (containing sialic acid) or desialated (sialic acid removed, so-called asialo form). Distal GlcNAc may be fucosylated as in the Sialyl Lewis[x] moiety. "Gal" is a galactose molecule. "Man" is a mannose molecule. "GlcNAc" is the N-actylglucosamine terminal (S) moiety. A1GP glycoforms can be mono-, di-tri- or quatra-antennary structures or "trees" attached to the P backbone or protein backbone.
The term stem cells includes, but is not limited to, mesenchymal stem cells and CD34+ cells.
The term "part of the wells" shall be understood that at least one well, preferably at least three wells should be filled. Preferably at least three wells must remain unfilled. Most preferably, half of the wells should be filled with $\alpha$-1 acid

glycoprotein and half left unfilled. Preferably, unfilled wells are covered with a buffered saline solution.

The α-1 acid glycoprotein solution is preferably a buffered solution of α-1 acid glycoprotein in physiological saline.

The higher the temperature, the shorter the incubation period of the matrix with the α-1 acid glycoprotein solution should be. For example, at 37°C the incubation period may be 1 hour; at room temperature - 3h; and at a temperature of 4°C - overnight, with the most favorable temperature being around 4°C.

Matrices with wells made of plastic, e.g. modified polystyrene, are preferably used. Typically, such matrices have 96 wells, but a different number of wells is also possible. Black matrices are most preferably used.

Before performing the test, the cells are cultured in an appropriate culture medium.

Fluorescent dyes are used to label cells. Preferably, highly sensitive dyes for the quantification of matrix-bound cells suitable for use in microplate fluorescence readers should be used.

Buffered saline (PBS) or physiological saline solution is preferably used for washing. Preferably, washing should be done twice or thrice. Automatic, semi-automatic and/or manual washings are used, with semi-automatic washing being the most preferred. Preferably, the matrix is washed three times after incubation with the α-1 acid glycoprotein solution, and twice after incubation with stem cells.

Stem cells are suspended in physiological saline (0,9% NaCl).

A diagnostic assay kit for determining the ability of stem cells to bind to mammalian heart tissue may additionally comprise solutions for preparation of α-1 acid glycoprotein solution and stem cells suspension, as well as fluorescent dyes.

The method according to the invention allows for the determination of the level of binding of stem cells to matrices, including both immortalized and primary stem cells.

The cells are placed on a properly prepared matrix, incubated with it for an appropriate time, and then unbound cells are removed by washing the matrices several times. Detection is based on measuring the green fluorescence signal from cells bound to the matrix, which are previously (before adding to the matrix wells) labeled with a fluorescent dye. This is a quantitative determination - the level of cell binding expressed as % binding relative to the negative control, i.e. wells without protein.

Thus, the invention will allow to determine whether the stem cells of a given patient, which could potentially be transplanted, will have a high potential to bind to the cardiac endothelium, covered with α-1 acid glycoprotein previously administered to the patient. This is due to the fact that α-1 acid glycoprotein increases cell retention in the heart, while the matrix, according to the invention, binds α-1 acid glycoprotein in a manner similar to the binding of this protein by the myocardial endothelium, which allows for the analysis of the *in vivo* behavior of cells in an in *vitro test.*

EXAMPLES

Example 1

In the black matrices with 96 wells made of polystyrene with three different types of modifications (Polysorp, Medisorp, Maxisorp), 50% of the wells were filled with a solution of α-1 acid glycoprotein in buffered saline solution (PBS) with a concentration of 50 μg/ml, in each of these 100 μl of protein solution was placed in the wells. The remaining wells were coated with PBS only. The prepared matrices were incubated at 4°C overnight or at 37°C for 1 h.

Then, the whole was washed three times with PBS solution in a semi-automatic manner, and then all wells were filled with three different volumes (50 μl, 75 μl or 100 μl) of MSC suspension in physiological saline ($10^3$ cells for each well), labeled with a fluorescent dye (calcein AM ( 2.5 μM)). The matrices were incubated for 1 hour at 37°C. After incubation, each matrix was washed twice with PBS in a semi-automatic manner and the level of green fluorescence was measured using an Infinite 200 Pro plate reader (Tecan).

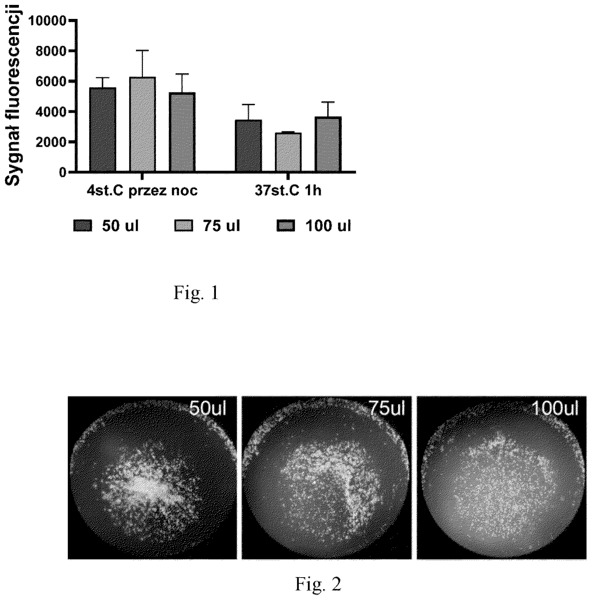

The obtained results indicate that MSC bind to the wells of the matrices, with higher level of binding for matrices coated with α-1 acid glycoprotein overnight at 4°C, when compared to the coating for 1 h at 37°C, regardless of the volume of cell suspension applied onto individual wells (Fig 1).

Fig. 1 - Analysis of the fluorescence signal from MSC bound to exemplary matrix wells previously coated with α-1 acid glycoprotein. Data for three volumes of MSC suspension applied onto individual wells. The fluorescence signal was measured after washing the wells with PBS solution.

Example 2

In the black 96-well modified polystyrene matrices, 50% of the wells were filled with a 50 μg/ml solution of α-1-acid glycoprotein in PBS. 100 μl of protein solution was placed in each of these wells. The remaining wells were coated with PBS only. The prepared matrices were incubated at 4°C overnight.

Then, the whole was washed three times with a PBS solution in a semi-automatic manner, and then all wells were filled with three different volumes (50 μl, 75 μl or 100 μl) of MSC suspension in physiological saline ($10^3$ cells for each well), labeled with a fluorescent dye (calcein AM ( 2.5 μM)). The matrices were incubated for 1 hour at 37°C. After incubation, each matrix was washed twice in a semi-automatic manner and observed in a fluorescence microscope in the green fluorescence channel.

The obtained results indicate that MSC placed in a suspension of 50 $\mu$l or 75 $\mu$l onto individual wells tend to aggregate in the central part of the well (Fig 2). In the case of a suspension with a volume of 100 $\mu$l, no such phenomenon was observed and the cells bound to the bottom of the wells in a more uniform manner. Thus, it is possible to perform the test with any volume of cell suspension, but the most preferred volume is 100 $\mu$l.

Fig. 2 - Microscopic analysis of the distribution of fluorescently labeled MSC bound to matrix wells. Representative images of matrix wells for three volumes of MSC suspensions applied onto individual wells, indicating cell aggregation in the center of the wells for 50 $\mu$l or 75 $\mu$l and no aggregation for the 100 $\mu$l suspension volume.

Example 3

In the black matrices with 96 wells made of polystyrene with three different types of modifications (Polysorp, Medisorp, Maxisorp), 50% of the wells were filled with a solution of acid $\alpha$-1 acid glycoprotein in PBS at a concentration of 10 $\mu$g/ml or 50 $\mu$g/ml. 100 $\mu$l of protein solution was placed in each of these wells. The remaining wells were coated with PBS only. The prepared matrices were incubated at 4°C overnight.

Then, the whole was washed three times with a PBS solution in a semi-automatic manner, and then all wells were filled with 100 $\mu$l of MSC cell suspension in physiological saline ($10^3$ cells for each well), labeled with a fluorescent dye (calcein AM (2.5 $\mu$M)). The matrices were incubated for 1 hour at 37°C. After incubation, each matrix was washed twice with PBS in a semi-automatic manner and the level of green fluorescence was measured using an Infinite 200 Pro plate reader (Tecan).

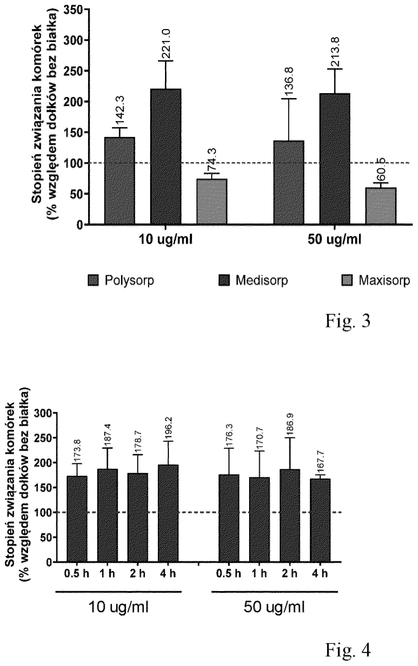

The obtained results indicate that, compared to wells covered only with the PBS solution, MSC were bound into each matrix type, with the degree of MSC binding being the highest for the Medisorp matrix. Additionally, there are no significant differences in the level of cell binding between the concentration of the $\alpha$-1 acid glycoprotein solution 10 $\mu$g/ml and 50 $\mu$g/ml. Therefore, a solution concentration of 10 $\mu$g/ml is sufficient for the test and is advantageous due to lower protein consumption (Fig. 3).

Fig 3. - Analysis of the degree of binding of MSC into wells of three types of matrices with different types of polystyrene modification, determined on the basis of the comparison of the level of fluorescence signal from cells bound to wells previously covered with $\alpha$-1 acid glycoprotein, to wells without protein (incubated only with PBS solution; 100%). Data for the two concentrations of $\alpha$-1 acid glycoprotein solution. The fluorescence signal was measured after washing the wells with PBS solution.

Example 4

In the four black 96-well Medisorp modified polystyrene matrices 50% of the wells were filled with a solution of $\alpha$-1acid glycoprotein in PBS at a concentration of 10 $\mu$g/ml or 50 $\mu$g/ml. 100 $\mu$l of protein solution was placed in each of these wells. The remaining wells were coated with PBS only. The prepared matrices were incubated at 4°C overnight.

Then, the whole was washed three times with a PBS solution in a semi-automatic manner, and then all wells were filled with 100 $\mu$l of MSC suspension in physiological saline (103 cells for each well), labeled with a fluorescent dye (calcein AM (2.5 $\mu$M)). The matrices were incubated for 30 min, 1 h, 2 h or 4 h at 37°C. After incubation, each matrix was washed twice in a semi-automatic manner and the level of green fluorescence was measured using an Infinite 200 Pro plate reader (Tecan).

The obtained results indicate that there are no significant differences in the level of MSC cell binding to the matrix wells between the tested incubation times. Therefore, a time in the range of 0.5-4 h is suitable for the test (Fig. 4). However, a short incubation time is beneficial to the overall speed of the test. Moreover, there were again no significant differences in the level of cell binding between the $\alpha$-1-acid glycoprotein solution concentration of 10 $\mu$g/ml and 50 $\mu$g/ml. Therefore, a solution concentration of 10 $\mu$g/ml is sufficient for the test and is advantageous due to lower protein consumption (Fig. 4).

Fig. 4 - Analysis of the degree of binding of MSC cells to the wells of a Medisorp-type modified polystyrene matrix, determined by comparing the level of fluorescence signal from cells bound to wells previously covered with $\alpha$-1 acid glycoprotein at two concentrations, compared to wells without protein (incubated only with PBS solution; 100% ). Data for four incubation times of MSC suspensions applied to individual wells. The fluorescence signal was measured after washing the wells with PBS solution.

Example 5

In 96-well black Medisorp-type modified polystyrene matrices, 93 wells were filled with solutions of $\alpha$-1-acid glycoprotein from two sources, obtained from human plasma, in PBS at a concentration of 10 $\mu$g/ml. 100 $\mu$l of protein solution was placed onto each of these wells. The remaining wells were coated with PBS only. The prepared matrices were incubated at 4°C overnight.

Then, the whole was washed three times with a PBS solution in a semi-automatic manner, and then all wells were filled with 100 μl of MSC suspension in physiological saline (103 cells for each well), labeled with a fluorescent dye (calcein AM (2.5 μM)). The matrices were incubated for 30 min or 1 h at 37°C. After incubation, each matrix was washed twice in a semi-automatic manner and the level of green fluorescence was measured using an Infinite 200 Pro plate reader (Tecan).

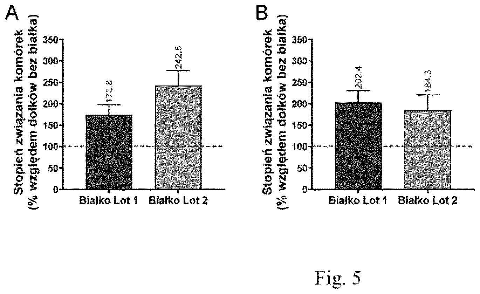

The obtained results indicate that there are no significant differences in the level of binding of MSC to matrix wells coated with α-1 acid glycoprotein from two different sources (Fig. 5). Furthermore, a short incubation time of 30 min (graph A) or 1 h (graph B) is beneficial for the overall speed of the test.

Fig. 5. Analysis of the degree of binding of MSC to the wells of a Medisorp-type modified polystyrene matrix, determined on the basis of a comparison of the level of fluorescence signal from cells bound to the wells previously covered with α-1 acid glycoprotein from two sources (Protein 1/2), comparing to the wells without protein (incubated only with PBS solution). Data for the incubation time of the MSC suspension applied onto individual wells for 30 min (A) or 1 h (B). The fluorescence signal was measured after washing the wells with PBS solution.

Example 6

Materials and reagents

> 96-well matrix Medisorp/ Microfluor 2 black (ThermoFisher Scientific; cat. 7805) with lid
> Pipette with tips (optimally multi-channel pipette)
> Falcon tubes and/or Eppendorf tubes
> Cell incubator (37°C, 5% $CO_2$)
> Centrifuge
> PBS (Thermo; cat. 14190-144)
> α-1 acid glycoprotein solution 10μg/ml in PBS
> 1mM Calcein AM in DMSO
> Culture medium: Alpha-MEM (ThermoFisher Scientific; cat. 22561054)
> Saline (0.9% NaCl)
> Matrix reader with fluorescence mode (e.g. Infinite 200 Pro plate reader (Tecan))
> Optionally: automatic matrix washer for PBS dispensing

Matrix coating

Perform all steps under the sterile conditions. Avoid drying out of wells.

> 1. Pre-wet matrix wells by adding sterile PBS w/o ions (200μl/well)
> 2. Discard liquid (by shaking it out) and place the matrix, wells side down, on clean paper wipes several times to remove all remaining liquid from wells
> 3. Add α-1 acid glycoprotein solution (10μg/ml) into wells (100μl/well) in triplicate per one tested condition
> 4. Add PBS into control (without α-1 acid glycoprotein) wells (100μl/well) in triplicate
> 5. Cover matrix with lid and incubate all night in 4°C
> 6. Following incubation, discard liquid (by shaking it out) and place the matrix, wells side down, on clean paper wipes several times to remove all remaining liquid from wells
> 7. Wash matrix by adding PBS into coated wells (200μl/well), then Discard liquid (by shaking it out) and place the matrix, wells side down, on clean paper wipes several times to remove all remaining liquid from wells
> 8. Repeat step 7 two times (to reach total 3 washings)
> 9. Proceed immediately with assay to avoid drying out of coated wells

Cell labelling and seeding

Start cell labelling prior to the washing out of matrix after coating with glycoprotein

> 1. Calculate the number of cells to be used for an assay, considering that $1 \times 10^4$ of cells is required per well (in triplicate per tested and control conditions). Include 10% excess of cells.
> 2. Prepare suspension of cells in culture medium to reach concentration of $0.5 \times 10^6$ cells/ml
> 3. Add 1mM Calcein-AM reagent to cell suspension to reach final concentration of 2. 5 μM

4. Place cell suspension in the incubator for 30 min. Mix cells gently every 10 minutes

5. Spin cells down (300g, 5min, room temperature) and carefully remove supernatant

6. Suspend cells in physiological saline to the final concentration of $1 \times 10^5$ cells/ml

7. Seed cells onto previously coated wells by adding 100μl of cell suspension ($1 \times 10^4$ cells) per well

8. Cover matrix with lid and place in the incubator for 1h to allow cell binding. Do not shake matrix during incubation

Removal of unbound cells and signal detection

1. Following cell binding, discard liquid (by shaking it out) and place the matrix, wells side down, on clean paper wipes several times to remove all remaining liquid from wells

2. Wash matrix by adding PBS into wells (200μl/well), then discarding liquid and blotting matrix few times onto clean paper wipes to remove all remaining liquid from wells

3. Repeat step 7 (to reach total 2 times of matrices washing)

4. Add physiological saline into wells (100μl/well). Include triplicate of control wells (covered with physiological saline solution only). Immediately read the fluorescent signal using matrix reader (e.g. 490 nm, em 520nm). Set well border zone for 500 μm and multiple reads per well mode (square filled type; 3 x 3 size)

Calculation of cell binding

1. Calculate mean value of fluorescent signal from well triplicates for tested conditions, control (without glycoprotein) and background control.

2. Subtract mean fluorescence of background control from mean for tested conditions and control (without glycoprotein), obtaining Meantest and Meancontrol, respectively.

3. Calculate MSCs binding efficacy (as % of control without glycoprotein) using the following formula:

$$\text{MSCs binding efficacy} = (\text{Mean}^{test} / \text{Mean}^{control}) \times 100\%$$

The performed test allows to determine whether a given patient's stem cells (MSC), which could potentially be transplanted, will have a high potential to bind to the cardiac endothelium, covered with $\alpha$-1 acid glycoprotein previously administered to the patient. This is due to the fact that $\alpha$-1 acid glycoprotein increases the retention of cells in the heart, while the matrix binds $\alpha$-1 acid glycoprotein in a way similar to the binding of this protein by the myocardial endothelium, which allows for reproducing the behavior of cells *in vivo* in an *in vitro* test.

## Claims

1. A method for the determination of the binding capacity of stem cells to mammalian heart tissue, **characterized in that**:

   (a) in the well matrix, some of the wells are filled with $\alpha$-1 acid glycoprotein solution and incubated;
   (b) after incubation, all wells are filled with a suspension of stem cells labeled with a fluorescent dye and incubated;
   (c) after incubation, the whole is washed;
   (d) the fluorescence level is then measured.

2. The method according to claim 1, **characterized in that** the mammal is a human.

3. The method of claim 1 or 2, **characterized in that** the $\alpha$-1 acid glycoprotein solution is $\alpha$-1 acid glycoprotein suspended in a buffered saline solution.

4. The method according to claim 1 or 2 or 3, **characterized in that** the concentration of the $\alpha$-1 acid glycoprotein solution is from 1 μg/ml to 1 mg/ml.

5. The method according to claim 1-4, **characterized in that** the volume of the $\alpha$-1 acid glycoprotein solution in each filled well is from 10% to 100% of the volume of the well.

6. The method according to claim 1-5, **characterized in that** the matrix is incubated with a solution of $\alpha$-1 acid glycoprotein for a 10 min to 48 hours at a temperature of 2 to 40 degrees Celsius.

7. The method according to claims 1-6, **characterized in that** after the time of incubation of the matrix with the $\alpha$-1 acid glycoprotein solution, the $\alpha$-1 acid glycoprotein solution is washed out by washing the matrix with a liquid in a volume ranging from 10% to 100% of the volume of the matrix well.

8. The method according to claim 1-7, **characterized in that** after washing the matrix, it is dried for a 1 minute to 12 hours at a temperature of 2 to 40 degrees Celsius in sterile conditions, and then covered with a lid adapted to the size of the matrix.

9. The method according to claims 1-8, **characterized in that** the concentration of stem cells in the suspension is from 10,000 cells/ml to 1,000,000 cells/ml.

10. The method according to claims 1-9, **characterized in that** the incubation period of the matrix with the stem cell suspension is from 1 minute to 24 hours at a temperature from 2 to 40 degrees Celsius.

11. The method according to claims 1-10, **characterized in that** after incubation, stem cells unbound to the matrix are washed away by washing the matrix with a liquid in a volume from 10% to 100% of the volume of the matrix well.

12. The method according to claims 1-11, **characterized in that** the matrix with bound stem cells (after rinsing off unbound cells) is filled with a liquid in a volume from 10% to 100% of the volume of each well of the matrix and fluorescence is measured.

13. A diagnostic assay kit for the determination of the binding capacity of stem cells to mammalian heart tissue, **characterized in that** it comprises: a matrix with wells and an $\alpha$-1 acid glycoprotein.

14. The diagnostic assay of claim 13, wherein the mammal is a human.

15. Use of $\alpha$-1 acid glycoprotein to determine the binding capacity of stem cells to mammalian cardiac tissues.

16. The use according to claim 15, **characterized in that** the mammal is a human.

17. The use according to claim 15 or 16, **characterized in that**:

   (a) the $\alpha$-1 acid glycoprotein solution is incubated;
   (b) after incubation, a suspension of stem cells labeled with a fluorescent dye is added to the solution and incubated;
   (c) after incubation, the whole is washed;
   (d) the fluorescence level is then measured.

Fig. 1

Fig. 2

Fig. 3

Fig. 4

Fig. 5

Europäisches
Patentamt

European
Patent Office

Office européen
des brevets

# EUROPEAN SEARCH REPORT

**Application Number**

EP 24 19 1023

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRAEYMAN N H ET AL: "A STUDY OF THE HETEROGENEITY OF HUMAN ALPHA1-ACID GLYCOPROTEIN WITH MONOCLONAL ANTIBODIES", HYBRIDOMA, LIEBERT, NEW YORK, NY, US, vol. 6, no. 6, 1 January 1987 (1987-01-01), pages 565-574, XP008058601, ISSN: 0272-457X * page 567, section "ELISA screening of Hybridoma Supernatants" * | 13,14 | INV. G01N33/50 |
| A,D | US 7 282 222 B2 (US DEPT VETERANS AFFAIRS [US]) 16 October 2007 (2007-10-16) * column 3, lines 24-27, column 4, lines 4 - 33, column 5, lines 30-45, column 6, lines 4 -51; example 6 * | 1-17 | |
| A | PARUL DIXIT ET AL: "Challenges in identifying the best source of stem cells for cardiac regeneration therapy", STEM CELL RESEARCH & THERAPY, BIOMED CENTRAL LTD, LONDON, UK, vol. 6, no. 1, 13 March 2015 (2015-03-13), page 26, XP021218649, ISSN: 1757-6512, DOI: 10.1186/S13287-015-0010-8 * the whole document * | 1-17 | TECHNICAL FIELDS SEARCHED (IPC) G01N |

The present search report has been drawn up for all claims

| Place of search | Date of completion of the search | Examiner |
|---|---|---|
| The Hague | 10 December 2024 | Adida, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 1023

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2024

| Patent document cited in search report | | Publication date | Patent family member(s) | | Publication date |
|---|---|---|---|---|---|
| US 7282222 | B2 | 16-10-2007 | AU | 2003225791 A1 | 29-09-2003 |
| | | | AU | 2003225793 A1 | 29-09-2003 |
| | | | AU | 2003267949 A1 | 31-12-2003 |
| | | | AU | 2009201808 A1 | 28-05-2009 |
| | | | CA | 2479309 A1 | 24-12-2003 |
| | | | CN | 1649621 A | 03-08-2005 |
| | | | EP | 1499347 A2 | 26-01-2005 |
| | | | HK | 1079987 A1 | 21-04-2006 |
| | | | JP | 2006501169 A | 12-01-2006 |
| | | | KR | 20050013531 A | 04-02-2005 |
| | | | US | 2004180040 A1 | 16-09-2004 |
| | | | US | 2006171934 A1 | 03-08-2006 |
| | | | US | 2007196331 A1 | 23-08-2007 |
| | | | US | 2007243173 A1 | 18-10-2007 |
| | | | US | 2007243174 A1 | 18-10-2007 |
| | | | US | 2007243175 A1 | 18-10-2007 |
| | | | US | 2007248577 A1 | 25-10-2007 |
| | | | US | 2010047215 A1 | 25-02-2010 |
| | | | WO | 03077864 A2 | 25-09-2003 |
| | | | WO | 03077865 A2 | 25-09-2003 |
| | | | WO | 03105908 A2 | 24-12-2003 |

For more details about this annex : see Official Journal of the European Patent Office, No. 12/82

**EP 4 685 484 A1**

**REFERENCES CITED IN THE DESCRIPTION**

**Patent documents cited in the description**

- US 7575938 B2
- EP 3816629 B1
- CN 105942966 A
- EP 1436615 A1
- WO 0039590 A3
- US 7358055 B2
- US 7282222 B2